# EUROPEAN PATENT APPLICATION

(11) **EP 1 908 417 A2**
(43) Date of publication of application: **09.04.2008**
(21) Application number: 07253948.9
(22) Date of filing: 05.10.2007
(51) Int. Cl.: A61B 17/072, A61F 2/00

(54) **Devices for reduction of post operative ileus**

(30) Priority: 06.10.2006 US 850056 P; 19.09.2007 US 856773
(71) Applicant: Ethicon Endo-Surgery, Inc., Cincinatti, OH 45242 (US)
(72) Inventor: Powell, Darrel M., Cincinnati, OH 45249 (US); Pomeroy, Mark, Cincinnati, OH 45241 (US); Murray, Michael A., Bellevue, KY 41073 (US); Shurtleff, Carl J., Mason, Ohio 45040 (US)
(74) Representative: Tunstall, Christopher Stephen

(57) **Abstract**

An apparatus and method for reducing post-operative ileus and/or gastric stasis is described. The method can include applying to the intestine a therapeutically effective amount of a composition comprising a drug that is effective in reducing post-operative ileus and/or gastric stasis, such as by introducing the composition through a surgical access device, such as a trocar or endoscope. The apparatus can include a surgical fastener and a buttress comprising the composition.

## Description

### CROSS-REFERENCE TO RELATED APPLICATIONS

This Patent application claims priority to and incorporates by reference US Provisional Application Serial Number 60/850,056 filed October 6, 2006 in the names of Powell et al.

This Patent application cross references "Methods for Reduction of Post Operative Ileus" filed on even date herewith.

This Patent Application incorporates by reference US Patent Application 11/761,707 filed June 12, 2007 and US Patent Application 11/761,726 filed June 12, 2007.

### BACKGROUND

The present invention relates generally to methods and devices for administering an anti-inflammatory agent to reduce post-operative ileus.

Post-operative ileus ("POI") is a transient impairment of bowel motility often resulting after surgery. POI is a common cause in delaying the body's return to normal gastrointestinal ("GI") function. POI is often responsible for extended hospital stays because hospitals will not discharge a patient until after a bowel movement. POI may also be responsible for some post-surgical readmissions to the hospital. The duration of the resulting hospital stay varies with the anatomic location of the surgery, the degree of surgical manipulation, and the magnitude of inflammatory responses. When the surgery directly affects the GI track, the resulting POI is often more severe and takes longer to correct. The patient is at risk of suffering increased medical costs from the duration of the hospital stay, increased financial difficulty from missing work, and increased health risks from the possible additional complications POI can cause.

Traditional treatment of POI includes mobilization, administration of laxatives, fluid administration, surgical techniques, and prokinetic agents. Accordingly, there is a need for alternative approaches for treating POI.

### SUMMARY OF THE INVENTION

In one aspect, the present invention provides a device for use in reducing post-operative ileus and/or gastric stasis, such as by applying a therapeutically effective amount of a composition containing a drug that is effective in reducing post-operative ileus to a site internal to the body, such as the intestine.

In one embodiment, the apparatus comprises a drug effective to reduce post-operative ileus; and a device for applying the drug. The apparatus can comprise at least one surgical fastener, such as a surgical clip or surgical staple. The apparatus can also include a buttress comprising the drug. In one embodiment, the apparatus comprises a stapler, such as a linear or circular stapler.

In another embodiment, the device can comprise a sheet of material comprising the drug in an amount therapeutically effective to reduce post operative ileus. The sheet can be adapted to selectively apply the drug to a treatment area, such as by including one or more openings that permit the drug to selectively applied to desired portion(s) of an internal body structure (e.g. a portion of the outer surface of the intestine).

The following is a non-exhaustive list of embodiments of this invention that are or may be claimed.

1. A surgical fastening apparatus comprising: at least one surgical fastener; and a therapeutically effective amount of a composition effective in reducing post operative ileus.

2. The apparatus of embodiment 1 wherein the device comprises at least one surgical staple.

3. The apparatus of embodiment 1 comprising a buttress.

4. The apparatus of embodiment 3 wherein the composition is operatively associated with the buttress.

5. The apparatus of embodiment 3 wherein the buttress is formed from a bioabsorbable material.

6. The apparatus of embodiment 1 wherein the surgical fastening apparatus is a surgical stapler.

7. The apparatus of embodiment 6 wherein the stapler is a linear stapler.

8. The apparatus of embodiment 6 wherein the stapler is a circular stapler.

9. The apparatus of embodiment 8 wherein the circular stapler comprises a stapler body, an anvil head removably attached to stapler body, a first buttress associated with the stapler body; and a second buttress associated with the anvil head; and wherein at least one of the buttresses comprises the composition effective in reducing post operative ileus.

10. An apparatus for use in reducing post-operative ileus, the apparatus comprising: a drug effective to reduce post-operative ileus; and a device for applying the drug, wherein the device is sized and shaped to be introduced through a surgical access device, and wherein the device provides for selective application of the drug.

11. The apparatus of embodiment 10 wherein the device comprises a sheet having at least one opening, and wherein the drug is disposed on the sheet, such that the sheet can be applied to an interior portion of the body without the drug contacting a selected area of the body.

12. The apparatus of embodiment 10 including a sprayable powder comprising the drug.

13. The apparatus of embodiment 12 wherein the sprayable powder comprises ORC.

14. The apparatus of embodiment 10 wherein the device comprises a roll-on applicator.

15. The apparatus of embodiment 10 wherein the drug is selected from the group consisting of a non-steroidal anti-inflammitory drug, galantamine, and a mast cell degranulation inhibitor.

16. The apparatus of embodiment 10 including a gel comprising the drug.

Other aspects of the present invention will become apparent from the following description, the accompanying drawings and the claims.

### BRIEF DESCRIPTION OF THE DRAWINGS

Fig. 1 is a perspective view of one embodiment of the present invention depicting a sheet containing a pharmaceutical agent effective in reducing POI;

Fig. 2 is a perspective view of another embodiment of the present invention depicting a sheet with a plurality of voids containing a pharmaceutical agent effective in reducing POI;

Fig. 3 is a side elevational view of a laparoscopy instrument useful in positioning a sheet of surgical material containing a pharmaceutical agent effective in reducing POI;

Fig. 4 is a perspective view of another embodiment of the present invention including an adhesive;

Fig. 5 is a flowchart illustrating a method of applying a pharmaceutical agent effective in reducing POI to the outside membrane of the intestine using an endoscope;

Fig. 6 is an elevation view of an applicator for applying a pharmaceutical agent effective in reducing POI;

Fig. 7 is a top plan view of a buttress containing a pharmaceutical agent effective in reducing POI;

Fig. 8 is a perspective view of the bioabsorbable circular staple buttress with buttresses containing a pharmaceutical agent effective in reducing POI;

Fig. 9 is a flowchart illustrating a method of applying a pharmaceutical agent at an operation site using an anastomotic device;

Fig. 10 is a side perspective view of a linear-type surgical stapler fitted with a pharmaceutical agent effective in reducing POI; and

Fig. 11 is a cross-sectional view of a surgical staple coated with a pharmaceutical agent effective in reducing POI.

### DETAILED DESCRIPTION

The following detailed description is intended to be representative only and not limiting as to devices and methods for applying a pharmaceutical agent to the tissues of the intestine or abdominal cavity. Many variations can be devised by one skilled in this area of technology, which are included within the scope of the present invention. The following detailed discussion of the various alternative and preferred embodiments will illustrate the general principles of the invention, examples of which are illustrated in the accompanying drawings.

### Sheets of Surgical Material

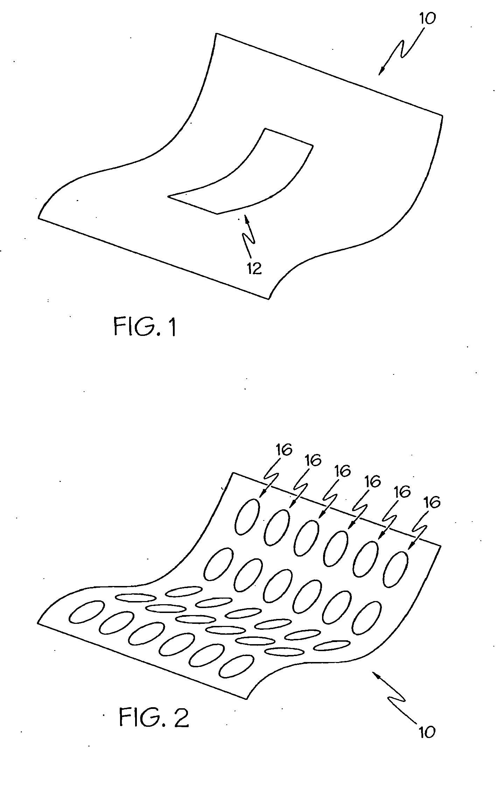

As illustrated in **FIG. 1,** one embodiment of the present invention is a sheet of surgical material **10** containing a pharmaceutical agent effective in reducing POI. In one embodiment, the pharmaceutical agent effective in reducing POI may be a NSAID. While the discussion herein will refer to NSAID's, those skilled in the art will understand that it also applies to other agents used to reduce POI. The NSAID composition to be used is to be applied in a pharmaceutically effective amount. The pharmaceutically effective amount will varying depending upon various factors including the type of surgery, the method and size of the incision, suturing, or stapling, the method used to apply the NSAID composition, and the form of the NSAID composition. The NSAID composition may be applied in the form of a sheet, foam, powder, sponge, slurry, liquid, spray, mesh, netting, polymer coating, gel, or the like. The NSAID composition typically is applied to the interior or exterior of the intestine.

Representative examples of NSAIDs (non-steroidal antiinflammatory agents) useful in this invention include but are not limited to propionic acid derivatives, acetic acid derivatives, fenamic acid derivative, biphenylcarboxylic acid derivatives, oxicams, and/or Cox2 inhibitors.

Representative examples of propionic acid derivatives include, but are not limited to, ibuprofen, naproxen, benoxaprofen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen and bucloxic acid. Structurally related propionic acid derivatives having similar cyclooxygenase inhibiting properties are also intended to be encompassed by this group. Presently preferred members of the propionic acid group include ibuprofen, naproxen, flurbiprofen, fenoprofen, ketoprofen and fenbufen.

Representative examples of acetic acid derivatives include, but are not limited to, indomethacin, sulindac, tolmetin, zomepirac, diclofenac, fenclofenac, alclofenac, ibufenac, isoxepac, furofenac, tiopinac, zidometacin, acemetacin, fentiazac, clidanac and oxpinac. Structurally related acetic acid derivatives having similar cyclooxygenase inhibiting properties are also intended to be encompassed by this group. Presently preferred members of the acetic acid group include zomepirac, diclofenac, indomethacin and sodium salts thereof.

Representative examples of fenamic acid derivatives include, but are not limited to, mefenamic acid, meclofenamic acid, flufenamic acid, niflumic acid and tolfenamic acid. Structurally related fenamic acid derivatives having similar cyclooxygenase inhibiting properties are also intended to be encompassed by this group. Presently preferred members of the fenamic acid group include mefenamic acid, meclofenamate acid, and sodium salts thereof.

Representative examples ofbiphenylcarboxylic acid derivatives include, but are not limited to, diflunisal and flufenisal. Structurally related biphenylcarboxylic acid derivatives having similar cyclooxygenase inhibiting properties are also intended to be encompassed by this group. Preferred members of this group are diflunisal and flufenisal.

Representative examples of oxicams include, but are not limited to, piroxicam, sudoxicam, isoxicam and CP-14, 304. Structurally related oxicams having similar properties are also intended to be encompassed by this group.

Representative examples of Cox2 inhibitors for use herein include those selected from the group consisting of Celecoxib, Rofecoxib, valdecoxib, Paracoxib and MK 663. Structurally related NSAIDs having similar Cox2 inhibiting properties are also intended to be encompassed by this group.

More particular examples of NSAIDS include 2-[(2,6-ldichlorophenyl) amino] benzeneacetic acid (also referred to as diclofenac), or 2-[(2,6-dichlorophenyl) amino]-benzeneacetic acid, monosodium salt, or 2-[(2,3-dimethylphenyl) amino]- benzoic acid (also referred to as mefenamic acid), and pharmaceutically acceptable salts of these compounds. It is understood that the present invention contemplates the use of not only the hereinbefore-described NSAIDs themselves, but also their pro-drugs which metabolize to the compounds and the analogs and biologically active salt forms thereof, as well as optical isomers which provide the same pharmaceutical results. Thus for purposes of this invention, the term "non-steroidal anti-inflammatory drug" is meant to include all derivatives or precursors such as salts, esters, pro-drugs, analogs, isomers, etc. which are NSAIDs themselves or which can yield materials which function as NSAIDs.

In another embodiment, the devices and methods of the present invention can employ galantamine for reduction of post operative ileus. A composition containing galantamine and effective for reducing post operative ileus can be applied to an internal body organ or body lumen (such as an outer surface portion of the patient's intestinal tract). The composition can be in any suitable form, such as, but not limited to, a gel or powder. Galantamine is described in US Patent 6,617,452 to Hille et al, and incorporated herein by reference herein.

The sheet of surgical material **10** may be any of a variety of surgical sheet material including oxidized regenerated cellulose ("ORC"), animal pericardium, polyglycolide, polyglycolic acid felt, caprolactone glycolide, polytetraflouroethylene, or other absorptive material that are capable of retaining a pharmaceutical agent effective in reducing POI. The pharmaceutical agent effective in reducing POI is preferably a NSAID. The ORC that may be used includes but is not limited to Interceed® absorbable adhesion barrier, Surgicel® absorbable hemostat, Surgicel Nu-Knit® absorbable hemostat, and Surgicel® Fibrillar absorbable hemostat (all available from Gynecare Worldwide or Johnson & Johnson Wound Management Worldwide, divisions of Ethicon, Inc., Somerville, New Jersey, a Johnson & Johnson Company), as well as Oxycel® absorbable cellulose surgical dressing (Becton Dickinson and Company, Morris Plains, New Jersey).

In one embodiment the NSAID may be incorporated into the sheet containing the delivery agent, in one embodiment an ORC, by any suitable method including but not limited to (a) dip coating the ORC fabric or textile in a solution or suspension of the NSAID, (b) spray coating a solution or suspension of the NSAID onto the ORC fabric or textile, (c) laminating at least one polymeric film formed from an aqueous, non-aqueous, or organic solution or suspension of NSAID onto the ORC fabric or textile, or (d) forming microporous polymeric matrix with NSAID and ORC powder or textile.

The sheet of surgical material **10** may also benefit from having a variety of other substances included such as stabilizers, wetting agents, or preservatives. In one embodiment other drugs may optionally be added to the composition, so long as it is compatible with the pharmaceutical agent effective in reducing POI and the remaining ingredients. These drugs include but are not limited to antibiotics, antiviral, anti-fungal, and anti-microbial agents.

In one embodiment the sheet of surgical material **10** containing a pharmaceutical agent effective in reducing POI may contain a void **12** as shown in **FIG. 1**. The void **12** may be an area of lower density ORC, lower concentration NSAID, or an opening cut into the sheet of surgical material **10**. The void **12** may be any geometric shape. Preferredly, the geometric shape of the void **12** is such that the sheet of surgical material **10** may be applied locally near the incision to administer an effective amount of NSAID at a desired location. The void **12** may be aligned with the patient's incision so the NSAID does not contact the incision, so as not to alter the body's normal healing process.

**FIG. 2** shows a sheet of surgical material **10** containing a pharmaceutical agent effective in reducing POI with a plurality of voids **16**. The plurality of voids 16 may be formed by varying the density of ORC and/or varying the concentration of NSAID across the sheet of surgical material **10** resulting in areas of little or no NSAID and/or ORC. The plurality of voids **16** may be formed by cutting openings into the sheet of surgical material. The plurality of voids **16** may be any geometric shape, and the geometric shapes may vary across the sheet of surgical material **10**. In one embodiment the plurality of voids **16** may be so numerous as to cause the sheet to function as a mesh.

The sheets of surgical material **10** containing the pharmaceutical agent effective in reducing POI shown in **FIG. 1-2** may be attached to the desired location within the abdominal cavity or the intestine using, staples, clips, sutures, or the like, or the sheet material may be self-adhesive, such as ORC.

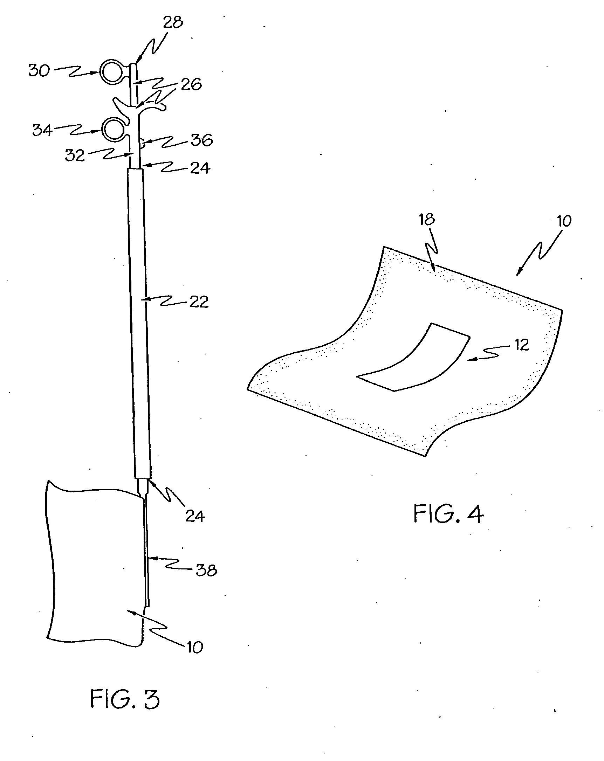

As illustrated in **FIG. 3,** a laparoscopy instrument **20** as described in United States Patent 5,503,623, incorporated herein by reference, may be used in the method for reducing post-operative ileus and/or gastric stasis to apply the sheet of surgical material **10** containing the pharmaceutical agent effective in reducing POI to the intestine or abdominal cavity. The laparoscopy instrument **20** includes a tubular member **22** having a uniform cylindrical bore **24**, and an elongated inserter instrument **26** that can be placed through the abdominal wall. The elongated inserter instrument **26** is comprised of an external tubular member **32** and an internal tubular member **28**. Both the internal and external tubular members **28, 32** have a handle **30, 34** respectively for manipulating the member. The external tubular member **32** has a lever **36** that is used to articulate the grasping portion **38** into multiple angular positions.

To apply the effective amount of a pharmaceutical agent effective in reducing POI, a selected edge of the sheet of surgical material **10** containing the pharmaceutical agent effective in reducing POI is clamped securely within the grasping portion **38** and the surgeon then furls the sheet around the grasping portion **38**. The elongated inserter instrument **26** with the sheet of surgical material **10** containing the pharmaceutical agent effective in reducing POI furled around the grasping portion **38** may then by inserted through the tubular member **22** into the abdominal cavity. The sheet of surgical material **10** containing the pharmaceutical agent effective in reducing POI may then be unfurled inside the abdominal cavity and applied to the desired site for reducing POI.

The laparoscopy instrument **20** may also be used for the removal of a sheet of surgical material **10** containing a pharmaceutical agent effective in reducing POI. The above method of operating the laparoscopy instrument **20** is simply reversed such that the elongated inserter instrument is inserted into the abdominal cavity and while in the abdominal cavity a sheet of surgical material **10** containing a pharmaceutical agent effective in reducing POI is clamped into the grasping portion **38**, furled around the grasping portion **38**, and removed out through the tubular member **20**.

**FIG. 4** shows another embodiment of a sheet of surgical material **10** containing a pharmaceutical agent effective in reducing POI that has an adhesive substance **18** applied over at least a portion of a side of the sheet, using methods described in United States Patent application US2005/0182443, incorporated herein by reference. The adhesive substance **18** may be applied on a single portion or a plurality of portions of the sheet of surgical material **10**, and may be applied in either a continuous or discontinuous manner. The adhesive substance **18** may be any suitable adhesive, but preferably, the adhesive substance **18** is a medical grade adhesive that is compatible with the pharmaceutical agent effective in reducing POI and any other compounds or ingredients in the composition. For example, the adhesive substance **18** may be an acrylic based pressure sensitive adhesives, rubber based pressure sensitive adhesives, silicone pressure adhesives, mixtures thereof, or the like as taught in United States Patent application US2005/0182443.

### Powder Mixtures.

In another embodiment the pharmaceutical agent effective in reducing POI may be applied to the abdominal cavity or the intestine as a powder to reduce post-operative ileus. In one embodiment, the composition includes a carrier such as ORC. Possible ORC compounds are listed above. The ORC is cryogenically milled into a powder of the desired particle size and then dry blended with the desired pharmaceutically effective amount of a NSAID, and stored under appropriate conditions to keep the powder dry. The powder containing the ORC and a NSAID may also by made as taught in United States Patent application US2005/0272697, incorporated hererin by reference, by chemically precipitating the desired composition, drying the composition, milling the composition using standard milling techniques to eliminate clumps and reduce particle size. Then the milled composition is sifted through sieves of varying size screen openings until the desired particle size is achieved.

Such a powder may be applied to the mucosa or serosal layer of the intestine. The powder may be sprayed using a sprayer similar in form or effect to any of the various sprayers taught in United States Patent application US2005/0220721, incorporated herein by reference. For example, the sprayer may be a handgrip sprayer that includes a squeeze bulb positioned in the sprayer's grip that has an inlet valve and a conduit connected to a powder reservoir, a standard aerosol canister that includes a pressurized gas and an internal reservoir capable of holding a powder, a squeeze bulb sprayer with a powder reservoir positioned such that it may be inserted and removed without inverting the sprayer device.

The powder composition containing a pharmaceutical agent effective in reducing POI may be introduced transanally, transorally, transgastrically, or any combination thereof using such a sprayer. One embodiment of the method of applying the pharmaceutical agent effective in reducing POI as a powder may be by spraying the intestinal mucosa layer using any of the various sprayers fitted with a flexible tube of the necessary length to reach the desired location within the patient where the powder is to be applied.

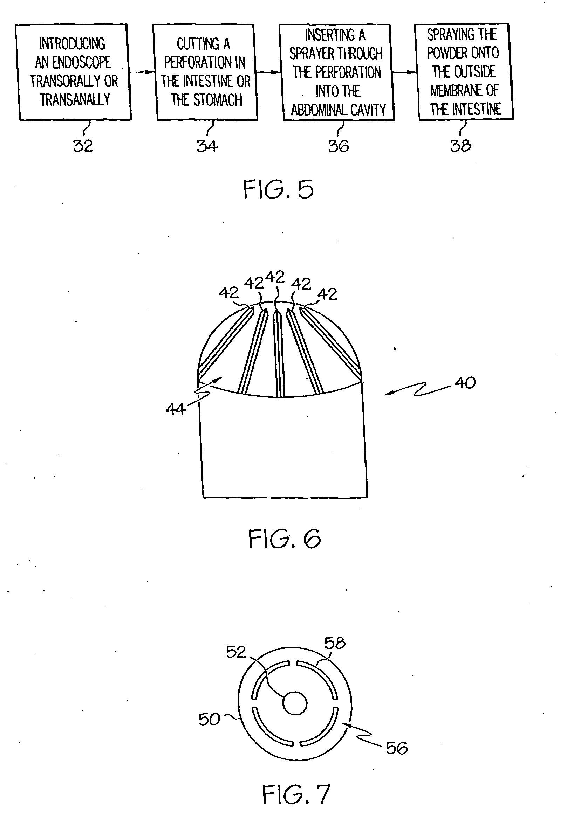

One method of applying the powder using the sprayer is shown in **FIG. 5.** An endoscope is introduced transorally or transanally **32** into the intestine or stomach. A perforation is cut into the intestine or the stomach **34.** Generally, when an endoscope is introduced transorally the perforation is cut into the stomach and when introduced transanally the perforation is cut into the intestine. The endoscope can be moved through the perforation and provide a channel for allowing other instruments to pass through the perforation as taught in United States patent 5,297,536, incorporated herein by reference. A sprayer, as described above, may be inserted through the perforation into the abdominal cavity **36.** The sprayer may need to be fitted with a long flexible tubing to reach the operation site or the site to be treated to reduce POI. A powder containing a NSAID composition, as described above, may be sprayed into the abdominal cavity and onto the outside membrane of the intestines **38.** In one embodiment, the sprayer's flexible tubing may be used through a working channel of the endoscope, a channel adjacent to the endoscope, or attached to a guide rail or tube attached to the endoscope.

### Gels and Slurries

The composition containing a pharmaceutical agent effective in reducing POI may be formed into a gel, slurry, bead, sponge, or the like. A gel, slurry, bead, or sponge containing a pharmaceutical agent effective in reducing POI may be applied to the interior or the exterior of the intestine in a manner consistent with those described for powders by using an instrument that can pump or push the composition to the desired location within the patient. United States patent 6,251,063, incorporated herein by reference, teaches a delivery method and gun or syringe that can be used to introduce a gel, slurry, bead, sponge, or the like. The needle tip of the gun or syringe may be fitted with a wider blunt tip as needed to apply the varying thickness and consistencies for the gel, slurry, bead, sponge, or the like. Additionally, using the method taught in United States patent 6,251,063 the compositions may be injected into the wall of the intestine to reduce POI.

FIG. 6 shows a roll-on applicator **40** that may be filled with a gel or slurry composition containing a pharmaceutical agent effective in reducing POI. Using the roll-on applicator **40** the NSAID may be applied to the desired location of the intestine to reduce POI. The roll-on applicator **40** could be smooth, or in one embodiment be provided with multiple channels or groves, shown as v-shaped grooves **42** in the surface of the applicator tip **44.** The grooves or channels **42** need not be v-shaped, but may assume any desired shape so long as the function of channeling the gel or slurry composition containing a NSAID to the application area is achieved. For example, the grooves or channels **42** may be v-shaped, u-shaped, square, semi-circular, semi-oval, or any other geometric shape. The grooves or channels **42** may be formed such that they are exposed on the surface, or they may be formed to be located beneath the surface of the applicator tip **44.** Additionally, the applicator tip **44** need not be dome-shaped, as shown in **FIG. 6**, but may be any suitable applicator tip shape that applies the gel or slurry composition containing a NSAID to the area to be treated for POI or gastric stasis.

The roll-on applicator may be used in laparoscopic surgery, open surgery, or the like. In another embodiment a laparoscopic device with a rigid or flexible shaft of varying length may be fitted with the roll-on applicator. The laparoscopic device may be used to reach into the patient's body to apply the NSAID at the desired location.

### Methods of applying Compositions using Surgical Devices

Another embodiment of the method of reducing post-operative ileus and/or gastric stasis applies a pharmaceutical agent effective in reducing POI as a composition on the buttress material used in a surgical procedure or has the buttress material made of the composition. In one embodiment, the pharmaceutical agent effective in reducing POI may be a NSAID. The design of the buttress material and the configuration of the NSAID composition on or within the buttress will vary with the surgical instrument that applies the buttress, the area to be treated, the patient being treated, and other medical variables.

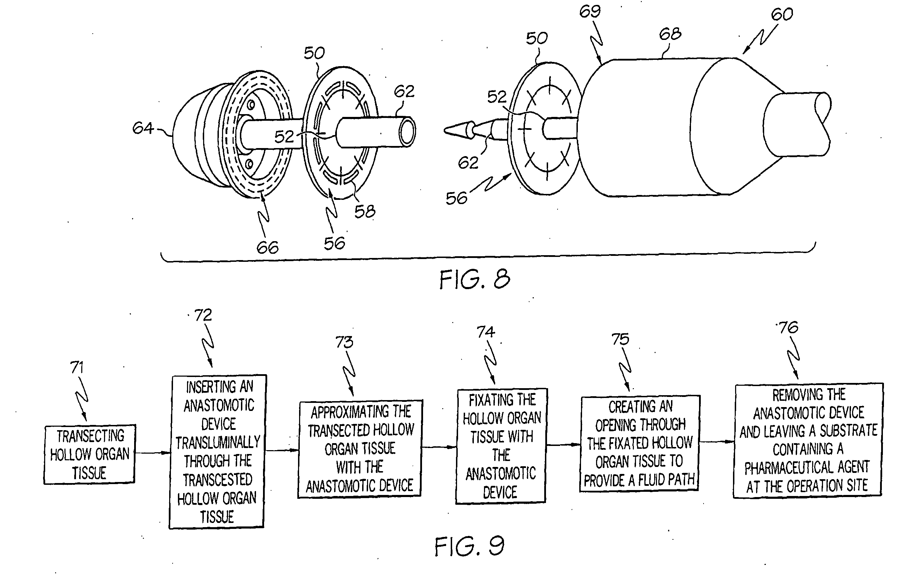

**FIG. 8** is a perspective view of a typical circular surgical stapler **60** with two stapler buttresses **50** mounted on a central shaft **62** as described in United States patent application US 2005/0059996, incorporated herein by reference. The stapler has an anvil head **64** with a staple compression surface **66**. As **FIG. 8** shows, the anvil head **64** is removably attached to the stapler body **68** via the central shaft **62.** The stapler body also has a compression surface **69** through which staples are ejected. One or both buttresses **50** may contain an NSAID presenting surface **56**. When the buttresses 50 are placed on the central shaft **62,** the NSAID presenting surface **56** will be facing away from the staple compression surface **66** and away from the staple body compression surface **69** respectively if both buttresses **50** are used. If the buttresses **50** are made of the NSAID composition, then any orientation of the buttresses **50** on the central shaft **62** will provide for the application of an effective amount of the NSAID. When the circular surgical stapler **60** is fired the buttresses **50** will come together with the two pieces of intestine to be joined sandwiched between them (also known as completing an anastomosis between two hollow organs), which will put the NSAID into contact with the intestine.

One embodiment of buttress material **50** shown in **FIG. 7** is for use with a circular surgical stapler **60** as shown in **FIG. 8.** The buttress material **50** has a central opening **52** so the buttress material **50** will fit into the circular surgical stapler **60**. The buttress **50** contains a NSAID composition. The NSAID composition **58** shown in **FIG. 7** will allow the staples to be in the outer most ring of the buttress. In one embodiment the NSAID composition **58** is in the outer most ring and the staples are in the next closest interior ring. Another embodiment has the NSAID and the staples together in the same ring, even though some clinicians believe the NSAID may decrease the body's natural healing inflammation along the staple or incision line.

In one embodiment the buttress material **50** may include the NSAID in the overall composition of the buttress material **50..** The NSAID may be any of the compounds described above. The concentration of the NSAID within the buttress material **50** may vary across the buttress and may be arranged in any configuration necessary to treat the effected area.

In another embodiment the NSAID may be applied to the buttress material **50** using any technique that will effectively hold the NSAID to the buttress material. The NSAID may be applied to one or both sides of the buttress material 50. When the NSAID is applied to only one side of the buttress material **50**, that side is called the NSAID presenting surface **56**. The concentration of the NSAID on the buttress material **50** may vary across the buttress. The NSAID may cover all or part of the buttress material **50**, and may be any geometric shape or concentration necessary to treat the affected area of the patient for POI or gastric stasis and to conform to the surgical instrument applying the buttress.

**FIG. 9** shows a flowchart of a method for transecting hollow organ tissue **71** to fixate tissue together using an anastomotic device **74** containing a substrate capable of capturing a pharmaceutical agent effective in reducing POI that will be left at the operation site **76**. An anastomotic device is inserted transluminally through the transected hollow organ tissue **72**, and approximated to the transected hollow organ tissues **73.** The anastomotic device contains at least one mechanical fastener or other fixation mechanism for fixating the tissue and a substrate capable of capturing a pharmaceutical agent. The anastomotic device is then used to fixate the tissue **74.** An opening is created through the fixated tissue to provide a fluid path **75.** Then the anastomotic device is removed leaving the substrate containing the pharmaceutical agent effective in reducing POI behind at the operation site **76.**

In one embodiment, the substrate is an ORC like Interceed® or caprolactone/glycolide, but may be any of the delivery agents listed above. The substrate may be a sheet, mesh, buttress, or any other substrate that is compatible with the anastomotic device. The pharmaceutical agent effective in reducing POI is preferredly a NSAID and may be any of the NSAIDs listed above. More preferredly the NSAID is diclofenac or a pharmaceutically acceptable salt thereof.

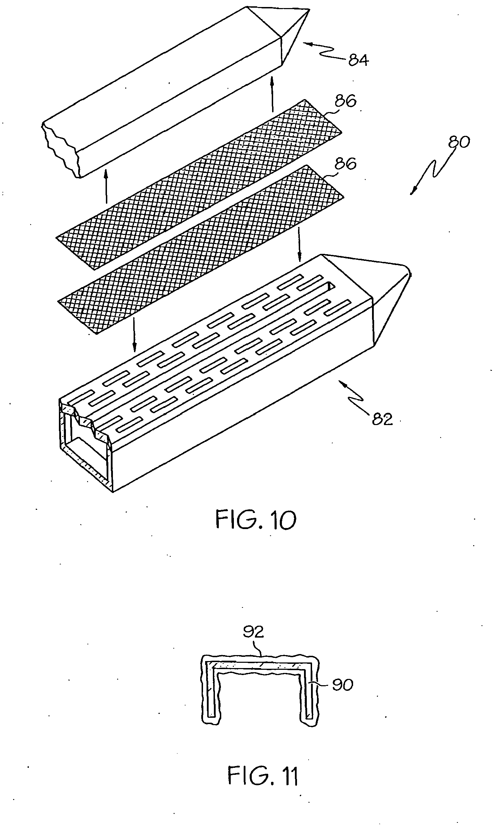

**FIG. 10** is a side perspective view of a linear-type surgical stapler **80** with a substrate **86** containing a pharmaceutical agent effective in reducing POI. The NSAID may be applied to the substrate **86** on one side, the NSAID surface **87**, both sides, or made part of the substrate material **86**. The linear-type surgical stapler **80** can utilize one or a plurality of the substrate **86**. The linear-type surgical stapler **80** has a staple cartridge **82** with a channel **88** therein, and an anvil **84**.

The substrate **86** containing a NSAID is positioned against the anvil **84** and/or staple cartridge **82** respectively with the NSAID surface **87** facing each other. The substrate **86** may be a sheet, mesh, or the like as described in **FIGS. 1-2, 4**. The substrate **86** may be buttress material or any other substrate material shaped to fit the linear-type surgical stapler **80**. In one embodiment, the substrate **86** is an ORC like Interceed® or caprolactone/glycolid, but may be any of the delivery agents listed above.

The sheet, mesh, buttress or substrate preferredly contain a NSAID as the pharmaceutical agent. The NSAID may be any of the compounds listed above, but more preferredly is diclofenac or a pharmaceutically acceptable salt thereof. The concentration of the NSAID may vary across the substrate **86** creating areas of low concentration and areas of high concentration. The NSAID may be concentrated in any geometric shape necessary to treat the effected area of the patient for POI or gastric stasis and to conform to the surgical instrument applying the substrate **86**. In one embodiment, the NSAID is concentrated in an area separate from the staples, but may be concentrated with the staples even though the NSAID may decrease the body's natural healing inflammation along the staple line.

**FIG. 11** shows a surgical staple **90** that has a coating **92** containing an effective amount of a composition containing a NSAID to reduce POI. In one embodiment, the surgical staple **90** may be made of a composition containing a NSAID or have the composition containing the NSAID impregnated into it. The staple may be an absorbable staple, a metal staple, or any other staple material capable of containing, holding, absorbing, or being coated with a NSAID. Preferably the composition containing the NSAID also contains a polymer, such as polyacrylic acid, hydroxylpropyl methyl cellulose, polyvinyl pyrrolidone, polyethylene glycol, and polyethylene oxide. The NSAID composition may also contain a variety of other substances such as stabilizers, wetting agents, or preservatives. Other drugs may also be added to the composition, so long as they are compatible with the NSAID and any other compounds or ingredients. These drugs include but are not limited to antibiotics, antiviral, anti-fungal, and anti-microbial agents.

### Packaging Kits

The pharmaceutical agent effective in reducing POI may be packaged as shown in United States patent 6,372,313, incorporated herein by reference. In one embodiment, the kit may contain the NSAID composition in a container, bottle, ampoule, pouch, sealed individual depressions, or the like of various sizes. The kit may also contain appliers of various sizes and shapes. The NSAID composition may be a solution capable of being placed onto an applier drop by drop. The NSAID may be a solution, slurry, or gel capable of having the applier dipped into the container and extracted containing a small amount of the NSAID ready for application to the patient's intestine or abdominal cavity. The NSAID may be a solid, powder, slurry, gel, solution, or the like stored in an ampoule that comes with an applier as shown in United States patent 6,340,097, incorporated herein by reference. The ampoule may have a shield that separates the NSAID composition from the air and must be broken by the applier to reach the composition.

The NSAID composition may need to be sterilized before use in treating POI. The NSAID composition may be packaged according to the methods in United States patent 6,412,639, incorporated herein by reference. The NSAID composition can be sterilized separately from the surgical tools and then remain sterile by separating the NSAID composition from the sterilization used for the surgical tools by way of a sterilization barrier built into the kit.

Although the present invention is shown and described with respect to certain aspects, various modifications will become apparent to those skilled in the art upon reading and understanding the specification and the appended claims. Further, each element described can be expressed as a means for performing the elements function.

## Claims

1. A surgical fastening apparatus comprising:
at least one surgical fastener; and
a therapeutically effective amount of a composition effective in reducing post operative ileus.

2. The apparatus of Claim 1 wherein the device comprises at least one surgical staple.

3. The apparatus of Claim 1 comprising a buttress.

4. The apparatus of Claim 3 wherein the composition is operatively associated with the buttress.

5. The apparatus of Claim 3 wherein the buttress is formed from a bioabsorbable material.

6. The apparatus of Claim 1 wherein the surgical fastening apparatus is a surgical stapler.

7. The apparatus of Claim 6 wherein the stapler is a linear stapler.

8. The apparatus of Claim 6 wherein the stapler is a circular stapler.

9. The apparatus of Claim 8 wherein the circular stapler comprises a stapler body, an anvil head removably attached to stapler body, a first buttress associated with the stapler body; and a second buttress associated with the anvil head; and wherein at least one of the buttresses comprises the composition effective in reducing post operative ileus.

10. An apparatus for use in reducing post-operative ileus, the apparatus comprising:
a drug effective to reduce post-operative ileus; and
a device for applying the drug, wherein the device is sized and shaped to be introduced through a surgical access device, and wherein the device provides for selective application of the drug.
